# EUROPEAN PATENT SPECIFICATION

(11) **EP 0 439 077 B1**
(45) Date of publication and mention of the grant of the patent: **17.05.2000**
(21) Application number: 91100616.1
(22) Date of filing: 18.01.1991
(51) Int. Cl.: C07K 14/15, G01N 33/569

(54) **Synthetic peptide compositions with immunoreactivities to antibodies to HTLV**
Immunaktive Peptidzusammensetzung gegen HTLV-Antikörper
Composition peptidique ayant une activité immunologique contre des anticorps contre HTLV

(30) Priority: 24.01.1990 US 469291
(43) Date of publication of application: 31.07.1991
(73) Proprietor: United Biomedical Inc., Lake Success New York 11042 (US)
(72) Inventor: Chang Yi Wang, New York 11021 (US)
(74) Representative: Vogeser, Werner, Dipl.-Ing.

(56) References cited:
- WO-A-89/08664
- WO-A-90/10231
- WO-A-90/15820
- US-A- 4 833 071
- JOURNAL OF IMMUNOLOGY vol. 142, no. 3, 1989, BALTIMORE pages 971 - 978; PALKER ET AL: 'Mapping of immunogenic regions of human T cell leukemia virus type 1 ( HTLV-1 ) gp46 envelope glycoproteins with env-coded synthetic peptides and a monoclonal antibody to gp46'

## Description

### INTRODUCTION

The present invention relates to synthetic peptide compositions with immunoreactivities to antibodies to HTLV

Human T-cell leukemia viruses have been linked to certain adult lymphoid malignancies, notably adult T-cell leukemia-lymphoma (ATL) and hairy T-Cell leukemia (HTL) (1-3, 24 and 25). There are two recognized subgroups, HTLV-I and HTLV-II. Up to the present, much of the work is directed to HTLV-I prevalent in ATL patients in Japan. However, recent studies show that the HTLV-II is more prevalent in the intravenous drug users in the metropolitan areas of U.S.A. (27, 28). Antibodies that react with HTLV proteins have been found in sera of ATL patients. These HTLV antibodies recognize both the gag core antigens and the envelope proteins of the viruses (4, 5, 27). Human Immunodeficiency Virus (HIV) is a retrovirus causatively linked to Acquired Immune Deficiency Syndrome (AIDS) and AIDS related complex (ARC). Antibodies that react with HIV proteins have been found in the sera of AIDS and ARC patients. These HIV antibodies recognize both the gag core antigens and envelope proteins of the HIV virus. In the United States, the disease AIDS is far more prevalent than ATL, with some individuals seropositive for HIV also being seropositive for HTLV.

The present invention relates to highly sensitive methods for the detection of antibodies to HTLV-I and/or HTLV-II in body fluids by the use of synthetic peptide compositions. The present invention further relates to a highly sensitive method for the simultaneous detection of antibodies to HTLV-I, HTLV-II and HIV in body fluids by the use of synthetic peptide compositions. One peptide composition comprises peptides having amino acid sequences corresponding to segments of the external (extracellular) portion of the HTLV-I and HTLV-II env protein, designated-gp46, and may further comprise peptides having amino acid sequences corresponding to segments of the transmembrane portion of the HTLV-I/HTLV-II env protein, designated gp21. These sequences have been found to be highly immunoreactive to antibodies in the sera of patients with ATL or HTL. Such peptide compositions are also useful for the production of a vaccine to prevent ATL or HTLV-II infection by stimulating the production of antibodies to HTLV-I/HTLV-II, which provide protection against HTLV-I/HTLV-II infection in healthy mammals, including humans. Furthermore, a peptide composition comprising peptides with amino acid sequences corresponding to portions of HTLV-I/HTLV-II envelope proteins may be used in conjunction with a peptide composition comprising peptides with amino acid sequences corresponding to portions of the HIV envelope and core proteins for the simultaneous detection of antibodies to HTLV-I, HTLV-II and HIV.

More specifically, the present invention is directed to peptide compositions, useful for the detection of HTLV-I and/or HTLV-II antibodies, which comprise peptides selected from the group consisting of chemically synthesized peptides containing about thirty-four, forty, thirty-eight, twenty, twenty-four and sixteen amino acids, or their analogues, in a prescribed sequence; analogues, segments, mixtures, conjugates and polymers thereof. The invention is further directed to the use of an HTLV-I and/or HTLV-II peptide composition in conjunction with an HIV peptide composition which comprises peptides selected from the group consisting of chemically synthesized peptides containing about twenty-one, nineteen, eleven and sixteen amino acids, sequence; analogues, segments, mixtures, conjugates and polymers thereof, for the simultaneous detection of antibodies to HTLV-I, HTLV-II and HIV in human body fluids. The present invention also provides a method for diagnosing HTLV infection, HTLV-I or HTLV-II infection.

The detection methods include an enzyme-linked immunoadsorbent assay (ELISA), multi-dot, multi-line, or multi-square blotting on nitrocellulose paper, and a passive hemagglutination assay using the peptides as the solid phase antigens. The preferred detection method is by ELISA.

### BACKGROUND OF THE INVENTION

The human T cell leukemia-lymphoma viruses (HTLV) are a family of related retroviruses originally isolated from patients with T cell lymphoma and cutaneous manifestations. A particular subgroup of the family, type I, now known as HTLV-I, has been causatively linked to malignancies which share clinical and epidemiologic features with the disease called adult T-cell leukemia-lymphoma (ATL) which occur in certain regions of Japan (6-9), the Caribbean Basin (10,11) and the southwestern United States (12). There are no known endemic areas for HTLV-II and no known causal relationship between any specific disease with HTLV-II. The source of HTLV-II virus introduced into the intravenous drug users is not known. Widescale seroprevalence studies for HTLV-II have not been carried out.

HTLV-II is structurally very similar to HTLV-I. The two viruses share approximately 50% sequence homology (29). HTLV-II was isolated from one patient who had hairy cell leukemia but no causal relationship was found. The amino acid sequence of the env protein of HTLV-II is identical to that of HTLV-I for 69% of the residues, and an additional 14% of the amino acids represent conservative substitutions (30,31). The X and pol genes are even more highly conserved than the env gene (32).

Because of the high degree of homology between HTLV-I and HTLV-II, standard testing assays by ELISA for HTLV-I based on whole viral lysate or recombinant proteins also cross react with HTLV-II. The peptides disclosed in U.S. 4,833,071 are also cross reactive with HTLV-II. No effective serological assay exists to distinguish between HTLV-I and HTLV-II env proteins although antigenic differences between the two viruses have been detected by neutralization of vesicular stomatitis virus pseudotypes (5). Two supplemental methods have been employed to confirm that antibodies to HTLV are present in samples that are shown to be reactive in an HTLV-I enzyme immunoassay. The Western Blot method for HTLV-I gives bands at p15, p19, p24, p28, p32, p36 and p55 for core proteins and at gp45 and gp61 for envelope proteins (32). The radioimmuno precipitation assay (RIPA) for HTLV-I gives bands for gp45 and gp61 for env proteins, p24 and p55 for core, and p40x for the X region (31). Neither tests, however, distinguish between the two viruses.

PCR has recently been used to distinguish between HTLV-I and HTLV-II. The PCR method provides definitive results (28). However, because of its exquisite sensitivity, it is subject to false positive results. Moreover, it is a very time consuming and expensive test.

Although the mechanism of transmission of HTLV-I is currently unknown, horizontal transmission of HTLV is clearly implicated by molecular and epidemiologic analyses (13,14). HTLV-I seropositivity in regions endemic for ATL is elevated overall in the general population and further elevated among close family members of patients and in the recipients of blood transfusions (15,16). HTLV-II seropositivity has been identified in intravenous drug users in the metropolitan areas of U.S.A. (27, 28).

This means that there is an urgent need for a safe, reliable and sensitive test to screen each blood sample before its inclusion in blood banks and to isolate blood donations derived from HTLV-I and/or HTLV-II infected individuals to avoid the inadvertent spread of the virus among patients who must receive blood transfusions, e.g. hemophiliacs and surgical patients.

There is an urgent need for a apid and less expensive method to distinguish between infection with HTLV-I and HTLV-II. Since 1988, mandatory screening of all donors for HTLV-I has been performed and donors reactive for HTLV-I, as well as HIV must be notified of their results. The uncertainty as to which virus, HTLV-I or HTLV-II, is responsible for seropositivity, renders it very difficult to counsel the donors accurately about their risk for contracting ATL or a neurological complication of HTLV-I. A method for distinguishing HTLV-I from HTLV-II is also important for seroprevalence studies to define endemic areas for HTLV-II and pathogenecity studies for both viruses (33).

The complete nucleotide sequence of the HTLV-I virus was reported in 1983 (17). This report elucidated the structure of the HTLV-I virus at both the DNA level and the predicted protein level and permitted further serological studies of different epitopes which may be present on the HTLV-I virus. The nucleotide sequence of the HTLV-II virus was reported in 1984, 1985 and 1986 (30, 31, 32).

Simultaneous to Seiki et al's report in 1983, Dr. Carl Saxinger at National Cancer Institute reported the use of the isolated HTLV-I virus as a solid-phase immunoadsorbent for the development of an enzyme immunoassay for the detection of HTLV-I antibodies in the African population (18).

It was further reported by Samuel et al. (19) that a combined cloning and expression system in E. Coli had been used to identify HTLV-I DNA encoded glycoproteins which reacted immunologically with antibodies in sera from ATL patients. HTLV-I DNA encoding the envelope protein was cleaved into fragments and inserted into an expression vector. The expression vectors were introduced into an E. Coli host by transformation. One clone, designated as pKS400, produced an envelope protein product found to be suitable for use as an immunoadsorbent to screen a group of 28 coded sera. Antibodies that recognized the bacterially synthesized HTLV-I envelope protein sequences were found in all sera that had been shown to have antibodies to HTLV by an ELISA assay with disrupted virions as the antigen (18).

Slamon et al, Application No. PCT/US 85/01803, published on March 27, 1986 under Publication No. W086/01834, described polypeptides associated with immunogenic sites of HTLV-I as expression products of the X region of HTLV-I, a highly conserved region located between env and the 3' LTR of the virus. The proteins, with a molecular weight of between 37 kd and 40 kd, were cloned and expressed as fusion proteins in E. coli. The resulting products were purified and used in liquid phase immunoprecipitation tests to screen sera. The results indicated an accuracy of from about 77% to 87% (20). All of the above failed to distinguish between infection by HTLV-I or HTLV-II because of the antigens used to detect the immunoreactivity.

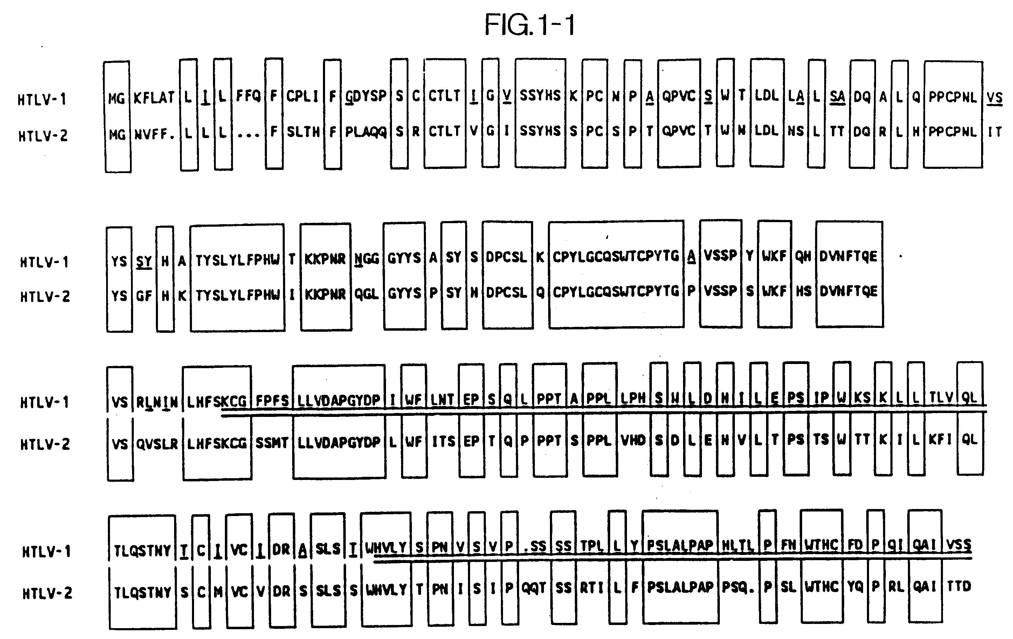

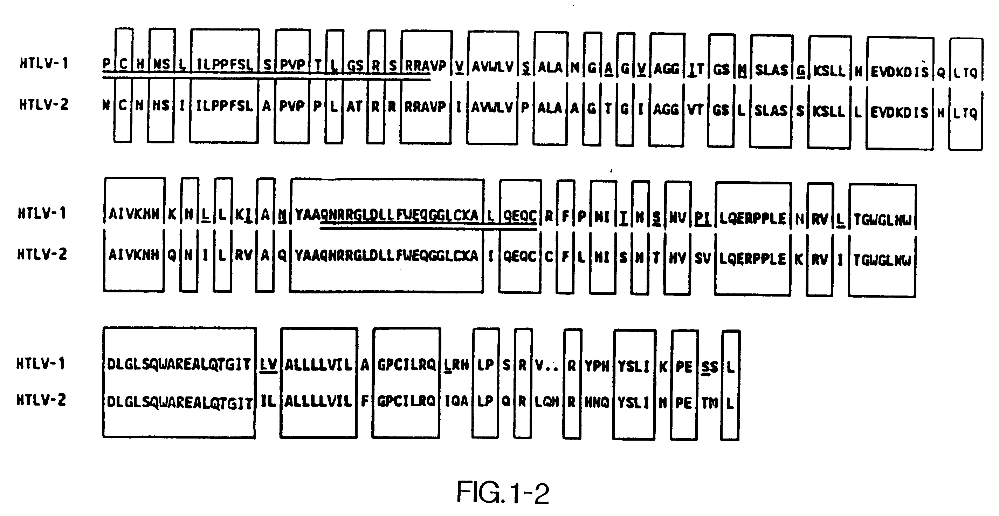

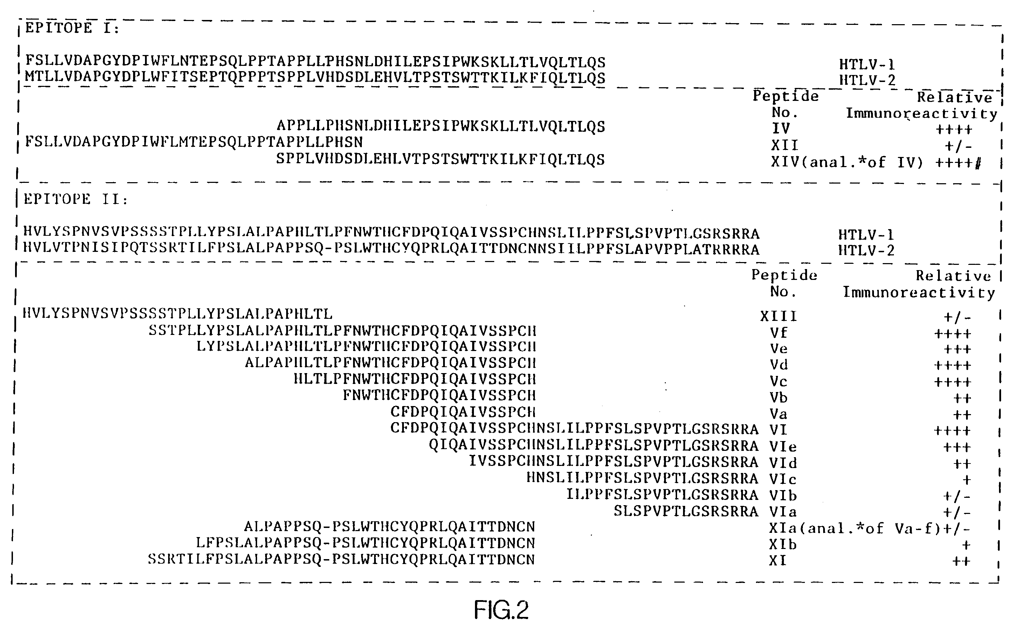

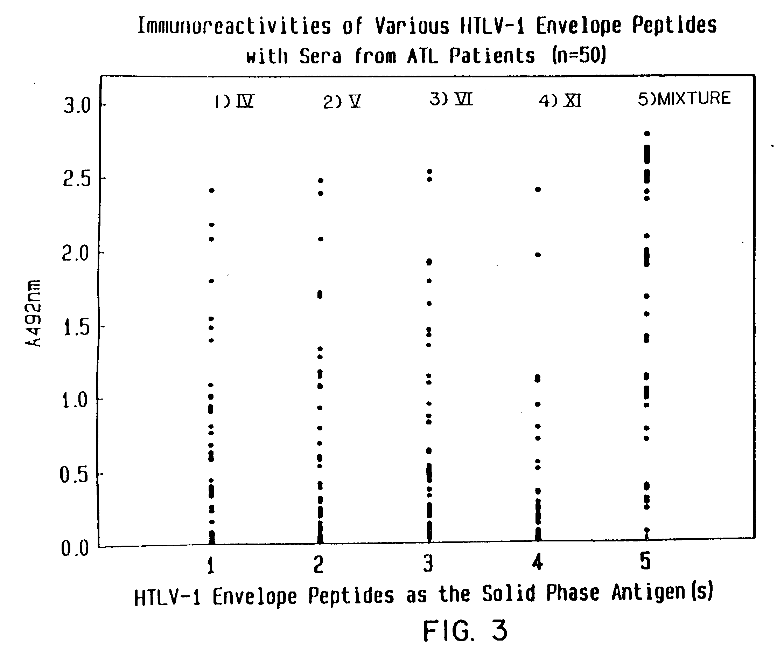

Synthetic peptides have been used increasingly to map antigenic or immunogenic sites on the surface of proteins and as possible vaccines. The named inventor and a colleague previously have taken this approach to identify and characterize highly antigenic epitopes on the envelope proteins of HIV and to develop sensitive and specific immunoassays for the detection of antibodies to HIV (previously designated HTLV-III) (21). See also U.S. Patent 4,735,896, issued April 5, 1988 and U.S. Patent 4,879,212 issued Nov. 7, 1989, the contents of which are, hereby, fully incorporated by reference. (22, 23). A similar approach is employed in this invention to select and identify highly antigenic epitopes in HTLV-I and HTLV-II. In selecting regions of the envelope protein for epitope analysis, several strategies were employed. First, regions that exhibited a relatively high conservation of amino acid sequence between HTLV-I and HTLV-II were sought. Second, multiple overlapping linear peptides covering whole regions of gp21, the transmembrane portion of the HTLV envelope protein (See Figure 1), were synthesized and characterized. Third, multiple overlapping linear peptides covering the whole region of gp46, the external portion of the HTLV envelope protein (See Figure 1), were synthesized and characterized. Three peptides, from the transmembrane portion, with the following sequences (See Figure 2), and a mixture thereof, were found to be highly immunoreactive with sera from patients with ATL:
GLDLLFWEQGGLCKALQEQC-NH2 (I)
QNRRGLDLLFWEQGGLCKALQEQC-NH2 (II)
NRRGLDLLFWEQGGLC-NH2 (III)
and three peptides, from the external portion, with the following sequences, and a mixture thereof, were also found to be highly immunoreactive with sera from patients with ATL (See Figure 3):
APPLLPHSNLDHILEPSIPWKSKLLTLVQLTLQS-NH₂ (IV)
SSTPLLYPSLALPAPHLTLPFNWTHCFDPQIQAIVSSPCH-NH₂ (V)
CFDPQIQAIVSSPCHNSLILPPFSLSPVPTLGSRSRRA-NH₂ (VI)
wherein:

| | |
|---|---|
| A= Ala = alanine, | G= Gly = glycine, |
| R= Arg = arginine, | I= Ile = isoleucine, |
| D= Asp = aspartic acid, | F= Phe = phenylalanine, |
| N= Asn = asparagine, | S= Ser = serine, |
| Q= Gln = glutamine, | W= Trp = tryptophan, |
| E= Glu = glutamic acid, | Y= Tyr = tyrosine, |
| L= Leu = leucine, | V= Val = valine, |
| K= Lys = lysine, | C= Cys = cysteine |
| H= His = histidine | P= Pro = proline |
| T= Thr = threonine | |

An example of an analogue peptide corresponding to Peptide IV of HTLV-I and found in the same region of HTLV-II contains the following sequence:
SPPLVHDSDLEHVLTPSTSWTTKILKFIQLTLQS-NH₂ (X)

Peptides I, II and III were described in the parent application which has now issued as U.S. Patent 4,833,071.

Assays for antibodies to HTLV-I and/or HTLV-II based upon chemically synthesized peptides show several advantages over assays utilizing whole disrupted virus or bacterially produced immunoadsorbents. The peptides can easily be synthesized in gram quantities by using automated solid-phase methods, thus providing a reproducible antigen of high integrity with consistent yields. Isolation of antigens from biological systems precludes such reproducibility. More importantly, non-specific reactivities seen in non-HTLV-I or non-HTLV-II infected individuals are likely due to the heterogeneity of the preparations used for assay. This is particularly true for assays using either the whole virus or Escherichia coli-derived recombinant products as immunoadsorbents. In these processes, the major histocompatibility antigens or endogenous bacterial proteins of the host cells are frequently copurified with the desired antigen virus or protein. Since antibodies to these contaminating antigens are frequently found in normal individuals, false-positive results cannot be eliminated by using current antigen isolation processes.

The assay of the present invention thus clearly eliminates the false-positive reactions encountered in the other methods and, at the same time, shows a high sensitivity to truly positive sera by the substantially increased signal-to-noise ratio. This increased signal-to-noise ratio likely results from the purity of the immunodsorbent. The assay of the present invention is also highly specific, in that peptide IV and its HTLV-II analogue (peptide X) are also found to be useful to distinguish between individual sera infected with HTLV-I or HTLV-II. That is to say, peptide IV preferentially detects antibodies to HTLV-I but not HTLV-II, and vice versa.

Furthermore, up to the present, no viable vaccine or method to provide protection against HTLV-I or HTLV-II infection has been reported. Utilization of deactivated virus provokes fears of contracting the disease, preventing its acceptability and use.

From Journal of Immunology, volume 142, No. 3, February 1, 1989, pages 971 to 978, the mapping of immunogenic regions of HTLV-I glycoproteins with synthetic peptides and a monoclonal antibody is known, wherein one peptide (4A) is overlapping with peptide IV of the present invention.

WO-A-89/08664 discloses synthetic peptide antigens corresponding to regions of the glycoprotein encoded by the ENV gene of HTLV-I. As evident these peptides are immunologically reactive with HTLV-I specific antibodies in assays for the detection of HTLV-I infection or exposure and in compositions to elicit the production of antibodies against HTLV-I in animals and man.

It is, therefore, an objective of the present invention to develop a detection or diagnostic procedure that does not require the use of the virus or lysates thereof as a test reagent.

A further objective is to develop a test procedure that is highly sensitive and accurate.

A further objective is to prepare a test reagent by chemical means. The synthetic reagent can then be used to detect the presence of antibodies to HTLV-I and/or HTLV-II in body fluids and diagnose ATL, thereby avoiding the danger of exposure to the virus or segments thereof and the unnecessary proliferation of the virus.

It is also an objective of the present invention to have a test reagent and procedure which can distinguish between HTLV-I and HTLV-II infection, to enable the medical profession to study the etiology of HTLV-II infection, the diseases caused by the HTLV-II virus, and its effect on the development of HIV infection in patients who are infected with both HIV and HTLV-II.

Another objective is to develop a vaccine which, when introduced into healthy mammals, including humans, will stimulate production of antibodies to HTLV-I, thereby providing protection against HTLV-I infection.

A further objective is to provide a non-viral immunogen which can be used in mammals for the development of monoclonal and polyclonal antibodies to HTLV-I.

Another objective is to develop a diagnostic procedure for the simultaneous detection of antibodies to HTLV (I and II) and antibodies to HIV.

### BRIEF DESCRIPTION OF THE INVENTION

According to the present invention, four additional peptides, each arranged in a specific sequence, have been made by solid phase peptide synthesis. These peptides have been found to be useful in a highly sensitive and accurate method for the detection of antibodies to HTLV-I/HTLV-II in sera and body fluids and in the diagnosis of ATL. Because of the high immunoreactivity, it is expected that the peptides are also useful in stimulating production of antibodies to HTLV-I/HTLV-II in healthy mammals such as Balb/c mice.

According to the present invention, a peptide composition useful for the detection of antibodies to HTLV-I/HTLV-II and diagnosis of ATL comprising a peptide
APPLLPHSNLDHILEPSIPWKSKLLTLVQLTLQS-X (IV)
   alone or in a mixture with any or both of
SSTPLLYPSLALPAPHLTLPFNWTHCFDPQIQAIVSSPCH-X (V)
   or
CFDPQIQAIVSSPCHNSLILPPFSLSPVPTLGSRSRRA-X (VI)
wherein X is -OH or -NH₂, analogues of said peptides having an amino acid sequence derived from a strain/isolate of HTLV in a region corresponding to the peptide and having specific immunoreactivity to antibodies to HTLV and conjugates and polymers of the peptide, wherein:

| | |
|---|---|
| A= Ala= alanine, | G= Gly= glycine, |
| R= Arg= arginine, | I= Ile= isoleucine, |
| D= Asp= aspartic acid, | F= Phe= phenylalanine, |
| N= Asn= asparagine, | S= Ser= serine, |
| Q= Gln= glutamine, | W= Trp= tryptophan, |
| E= Glu= glutamic acid, | Y= Tyr= tyrosine, |
| L= Leu= leucine, | V= Val= valine, |
| K= Lys= lysine, | C= Cys= cysteine. |
| H= His= histidine | P= Pro= proline |
| T= Thr= threonine | |

The highly sensitive and accurate method of detecting antibodies to HTLV-I/HTLV-II in body fluids and diagnosis of ATL comprises the following steps:
A. coating a solid support with an effective amount of a peptide composition comprising a peptide selected front the group consisting of:
   APPLLPHSNLDHILEPSIPWKSKLLTLVQLTLQS-X (IV)
      alone or in a mixture with any or both of
   SSTPLLYPSLALPAPHLTLPFNWTHCFDPQIQAIVSSPCH-X (V)
      or
   CFDPQIQAIVSSPCHNSLILPPFSLSPVPTLVSRSRPA-X (VI)
   wherein X is -OH or -NH₂, analogues of said peptides having an amino acid sequence derived from a strain/isolate of HTLV in a region corresponding to the peptide and having specific immunoreactivity to antibodies to HTLV and conjugates and polymers of the peptide
B. mixing a test specimen diluted with a buffer wherein the antibodies to HTLV in the test specimen form a peptide-antibody complex with the peptide
C. incubating the mixture; and
D. detecting the presence of the peptide-antibody complex.

Further, according to the present invention, the peptides by themselves, or when coupled to a protein or a polymeric carrier, or when polymerized to homo or hetero dimers or higher oligomers by cysteine oxidation, induced disulfide cross linking, or when polymerized to homo or hetero dimers or higher oligomers by use of homo or hetero functional multivalent cross linking reagents, or when directly synthesized onto a polyvalent lysine resin, can be used to stimulate production of antibodies to HTLV-I and/or HTLV-II in healthy mammals, including humans. The method comprises introducing an effective amount of the peptide composition including a mixture of these six peptides, conjugated to a carrier, such as human serum albumin, or as a polymer, into the body of a healthy mammal by intraperitoneal or subcutaneous injection.

Analogues of peptides I to VI can be found in HTLV-II. See Fig. 1. Such sequences are: 
GLDLLFWEQGGLCKAIQEQC-X (VII)
QNRRGLDLLFWEQGGLCKAIQEQC-X (VIII)
NRRGLDLLFWEQGGLC-X (IX)
SPPLVHDSDLEHVLTPSTSWTTKILKFIQLTLQS-X (X)
SSRTILFPSLALPAPPSQPSLWTHCYQPRLQAITTDNCN-X (XI)
CYQPRLQAITTDNCNNSIILPPFSLAPVPLATRRRRA-X (XII)
 (The differences from HTLV-I have been underscored.)

Peptides VII to XII are useful for detecting antibodies to HTLV-II in body fluids. A review of these sequences show that peptide IX is identical to peptide III; peptides VII and VIII are identical to peptides I and II except for one amino acid; whereas peptides X, XI and XII contain multiple sites where the amino acids are substituted according to the corresponding HTLV-II amino acids sequence and are, therefore, quite different from that in peptides IV, V and VI.

It has been found that peptide IV and X are specific for HTLV-I and HTLV-II respectively. That is, peptide IV is not as reactive to antibodies to HTLV-II and peptide X is not as reactive to antibodies to HTLV-I. Because of this, peptides IV and X may be used to distinguish between HTLV-I and HTLV-II seropositivity.

In addition, according to the present invention, mixtures of peptides IV-VI and X-XII may be used to detect the presence of HTLV-I/II in body fluids. Further, a peptide composition useful for the detection of antibodies to HTLV-I/HTLV-II may be used in conjunction with peptide compositions useful for the detection of antibodies to HIV-1 and HIV-2, for the simultaneous detection of infection by both HTLV-I and II and HIV-1 and HIV-2. Peptide compositions useful for the detection of antibodies to HIV-1 and HIV-2 comprise chemically synthesized peptides of the following amino acids, or their analogues, in the prescribed sequences wherein the sequence for HIV-2 is an analogue of peptide VII and peptide VIII:

### HIV-1

RILAVERYLKDQQLLGIWGCS-X (XIII)
IWGCSGKLICTTAVPWNAS-X (XIV)
IVRMYSPTSIL-X (XV)

### HIV-2

DQARLNSWGCAFRQVC (XVI)

wherein X is -OH or -NH₂, and include analogues and polymers are as defined above, wherein:

| | |
|---|---|
| A= Ala= alanine, | G= Gly= glycine, |
| R= Arg= arginine, | I= Ile= isoleucine, |
| D= Asp= aspartic acid, | F= Phe= phenylalanine, |
| N= Asn= asparagine, | S= Ser= serine, |
| Q= Gln= glutamine, | W= Trp= tryptophan, |
| E= Glu= glutamic acid, | Y= Tyr= tyrosine, |
| L= Leu= leucine, | V= Val= valine, |
| K= Lys= lysine, | C= Cys= cysteine. |
| H= His= histidine | P= Pro= proline |
| T= Thr= threonine | |
| M= Met= methionine | |

The underlined amino acids indicate the residues shared between various isolates. For HIV-2 peptide XVI, substitutions were made in the corresponding HIV-2 envelope protein amino acid sequence that would be predicted from the nucleotide sequence.

### BRIEF DESCRIPTION OF THE DRAWINGS

Figures 1-1 and 1-2 show and compare the amino acid sequences of the HTLV-I and HTLV-II envelope proteins.
Figure 2 shows the amino acid sequences of the chemically synthesized peptides described herein.
Figure 3 is a histogram depicting the immunoreactivties described herein, with sera from ATL patients.
Figure 4 is a histogram depicting the immunoreactivties of the peptides described herein with sera from patients with HIV infection, patients with ATL, and random blood donors.
Figure 5 is a histogram depicting the simultaneous detection of antibodies to HTLV-I and HIV (1 and 2) by an enzyme immunoassay employing a mixture of seven chemically synthesized peptides described herein.

### DETAILED DESCRIPTION OF THE INVENTION

In accordance with the present invention, six peptides have been chemically synthesized for the detection of antibodies to HTLV-I or HTLV-II in body fluids and the diagnosis of ATL, and for the vaccination of healthy mammals by stimulating the production of antibodies to HTLV-I or HTLV-II in healthy mammals. These peptides are arranged in the following sequences:
GLDLLFWEQGGLCKALQEQC-X (I)
QNRRGLDLLFWEQGGLCKALQEQC-X (II)
NRRGLDLLFWEQGGLC-X (III)
APPLLPHSNLDHILEPSIPWKSKLLTLVQLTLQS-X (IV)
SSTPLLYPSLALPAPHLTLPFNWTHCFDPQIQAIVSSPCH-X (V)
CFDPQIQAIVSSPCHNSLILPPFSLSPVPTLGSRSRRA-X (VI)
SPPLVHDSDLEHVLTPSTSWTTKILKFIQLTLQS-X (X)
wherein X is -OH or -NH₂.

These peptides may comprise analogues as defined above, i.e. longer or shorter peptide chains by having more amino acids added to the terminal amino acids, e.g., -Gly-,-Gln-,-Asn- and -Cys- of the above sequence, or by amino acids removed from either terminal end. For example, peptide X is an analogue of peptide IV.

These peptides may also comprise conjugates, i.e., they may be coupled to carrier proteins such as bovine serum albumin (BSA) or human serum albumin (HSA). Furthermore, these peptides may comprise polymers, i.e, they may be synthesized on a polymeric resin, such as a branching octameric lysine resin. It is expected that as long as the peptide immunoreactivities recognizable by the antibodies to HTLV-I/HTLV-II are preserved, analogues of the synthetic peptide may also comprise substitutions, insertions and/or deletions of the recited amino acids of the above sequence.

In addition, to accommodate strain-to-strain variations among different isolates, adjustments for conservative substitutions and selection among the alternatives where non-conservative substitutions are involved, may be made in the prescribed sequences. For example, peptides X to XII found in the HTLV-II strain may also be used.

The amino acid sequences of the polypeptides useful as test reagents for the detection of antibodies to HTLV-I or HTLV-II in body fluids and diagnosis of ATL are selected to correspond to a partial segment of the amino acid sequence of the HTLV virus designated as gp21, and to a partial segment of the amino acid sequence of the HTLV virus designated as gp46, both parts of gp61, which defines the envelope protein of the HTLV-I or HTLV-II virus.

The peptides useful as solid phase immunoadsorbents for the detection of antibodies HTLV-I were synthesized by the "classical" Merrifield method of solid phase peptide synthesis using side chain protected t-Boc-amino acids to correspond to the following amino acid sequences:
APPLLPHSNLDHILEPSIPWKSKLLTLVQLTLQS-X (IV)
SSTPLLYPSLALPAPHLTLPFNWTHCFDPQIQAIVSSPCH-X (V)
CFDPQIQAIVSSPCHNSLILPPFSLSPVPTLGSRSRRA-X (VI)
SPPLVHDSDLEHVLTPSTSWTTKILKFIQLTLQS-X (X)
wherein X is -OH or -NH₂.

Other analogues of these peptides can be prepared by varying the amino acid sequences either by adding, subtracting, substituting, or deleting desired t-Boc-amino acid(s).

For example, analogue peptides VII to XII having the following sequences can also be prepared.

GLDLLFWEQGGLCKAIQEQC-X (VII)
QNRRGLDLLFWEQGGLCKAIQEQC-X (VIII)
NRRGLDLLFWEQGGLC-X (IX)
SPPLVHDSDLEHVLTPSTSWTTKILKFIQLTLQS-X (X)
SSRTILEPSLALPAPPSQPSLWTHCYQPRLQAITTDNCN-X (XI)
CYQPRLQAITTDNCNNSIILPPFSLAPVPLATRRRRA-X (XII)

Following completion of assembly of the desired blocked peptide on the resin, the peptide-resin is treated with anhydrous hydrofluoric acid to cleave the peptide from the resin. Functional groups of amino acids which are blocked during synthesis by benzyl-derived blocking groups are also cleaved from the peptide simultaneously. The free peptide is then analyzed and purified by high performance liquid chromatography (HPLC) and characterized biochemically by amino acid analysis.

The peptides synthesized according to the above described procedure are highly reactive with antibodies to HTLV-I and/or HTLV-II and can be used as a highly sensitive and specific immunoadsorbent for the detection of the antibodies against HTLV-I and/or HTLV-II.

Fig. 3 show the data obtained with sera from ATL patients using an ELISA method wherein the well plates are coated with peptides IV, V and VI at 1.0 ug of each. Table I shows the data obtained with sera from ATL patients using an ELISA method wherein the well plates are coated with a mixture of peptides II, IV, V and VI, in a weight ratio of 1:0.25:1:1 (II:IV:V:VI). Table II shows the data obtained with sera from ATL patients utilizing an agglutination method wherein the red blood cells (RBC) are coated with a peptide VI-BSA conjugate. Tables III and IV show the unique and specific immuno reactivities associated with peptide IV and peptide X and their usefulness in differentiating between subjects with HTLV-I or HTLV-II infections.

Based on the high degree of sensitivity and specificity of the peptide compositions according to the present invention in the immunoreaction to antibodies to HTLV-I and/or HTLV-II, it is believed that the peptide compositions may also be useful as a vaccine for ATL and/or HTLV-II infection, and as immunogens for the development of both monoclonal and polyclonal antibodies to HTLV-I and/or HTLV-II in mammals, including humans. The peptide compositions when coupled to a protein or synthesized on a polymer carrier resin (e.g., an octameric lysine resin) or when polymerized to homo or hetero dimers or higher oligomers by cysteine oxidation, induced disulfide cross linking, or when polymerized to homo or hetero dimers or higher oligomers by use of homo or hetero functional multivalent cross linking reagents, can be introduced to normal subjects to stimulate production of antibodies to HTLV-I and/or HTLV-II, and provide protection against infection by HTLV-I and/or HTLV-II in healthy mammals. Since the peptide composition according to the present invention is not derived biochemically from the virus, there is no danger of exposing the normal subjects who are to be vaccinated to the disease.

The advantages of using the peptides according to the present invention are many.

The peptides are chemically synthesized. This means that there is no involvement with the HTLV-I or HTLV-II virus at any time during the process of making the test reagent or the vaccine. During the preparation of the vaccine or the vaccination process, production workers or individuals in the health professions do not risk exposure to the HTLV-I or HTLV-II virus. Further, up to the final step of the test to detect antibodies to HTLV-I or HTLV-II, where the test reagent is exposed to samples of sera or body fluid, there is no risk of exposure of the laboratory worker to the HTLV-I or HTLV-II virus.

Another problem which is avoided by the process of the present invention is the possibility of false positive results caused by the presence of antigenic materials from host cells co-purified with the HTLV-I or HTLV-II viral lysate preparation or E-Coli derived proteins co-purified with expressed viral fragments. Certain normal individuals have antibodies to E. Coli or human leukocyte antigens, e.g. HLA, which are cross reactive with the antigenic materials from host cells. Sera samples from these normal individuals may show a positive response in the ELISA or IRMA tests.

Further, with appropriate amino acid analogue substitutions, it is expected that various peptide analogues based on the prescribed amino acid sequence can be synthesized with properties giving rise to lower background readings or better adsorption capacity to solid phases useful for HTLV-I or HTLV-II antibodies screening assays.

Moreover, because the peptide compositions of the present invention are synthetically prepared, the quality can be controlled and as a result, reproducibility of the test results can be assured. Also, since very small amounts of peptides are required for each test procedure, and because the expense of preparing the peptides is relatively low, the cost of screening body fluids for antibodies to HTLV-I or HTLV-II, and diagnosis of ATL and/or HTLV-II infection and the preparation of a vaccine is relatively low.

The peptides prepared in accordance with the present invention can be used to detect HTLV-I and/or HTLV-II infection and diagnose ATL by using it as the test reagent in an enzyme-linked immunoadsorbent assay (ELISA), an enzyme immunodot assay, an agglutination assay, a radioimmunoradiometric assay (IRMA), or other well-known immunoassays. The preferred method is ELISA. The ELISA technique is exemplified in Examples 1 and 2, the IRMA technique is exemplified in Example 5, and the agglutination assay in Examples 3 and 6.

It is to be noted that in the following methods, 0.25% by weight of glutaraldehyde may be added in the coating buffer to facilitate better peptide binding onto the plates or beads. Further, horseradish peroxidase conjugated mouse monoclonal anti-human IgG antibody may be used in place of horseradish peroxidase conjugated goat anti human IgG as the second antibody tracer.

The gelatin used in these processes can include calf skin gelatin, pig skin gelatin, fish gelatin or any known available gelatin proteins or be replaced with albumin proteins.

In Example 10, it is shown that peptide IV is preferentially reactive to antibodies to HTLV-I and not reactive to HTLV-II and thus can be used to distinguish HTLV-I infection from HTLV-II infection.

Similarly, in Example 11, it is shown that peptide X, which is an analogue peptide derived from the amino acid sequence of HTLV-II, a variant of HTLV-I, is only reactive to antibodies to HTLV-II and can be used to specifically detect HTLV-II infection.

### EXAMPLE 1

### Detection of Antibodies to HTLV-I/HTLV-II by an Enzyme-Linked Immunoadsorbent Assay

Wells of 96-well plates were each coated at 4°C overnight (or 3 hours at room temperature), with each of the three peptides IV, V, VI prepared as described at 1.0 µg each per well per peptide in 100 µl 10mM NaHCO₃ buffer, pH 9.5. The wells were washed three times with phosphate buffered saline (PBS) and then incubated with 250 µl of 3% by weight of gelatin in PBS at 37°C for 1 hour to block non-specific protein binding sites, followed by three more washes with PBS containing 0.05% by volume of Tween 20®. The test sera (blood taken from a patient or normal individual) were diluted with PBS containing 20% by volume normal goat serum, 1% by weight gelatin and 0.05% by volume Tween® 20 at dilutions of 1:20 volume to volume, respectively. 200 µl of the diluted sera were added of each well and allowed to react for 1 hour at 37° C. The wells were then washed three times with 0.05% by volume Tween® 20 in PBS in order to remove unbound antibodies. Horseradish peroxidase conjugated goat anti-human IgG was used as a second antibody tracer to bind with the HTLV-I antibody-antigen complex formed in positive wells. 100 µl of peroxidase labeled goat anti human IgG at a dilution of 1:3000 in 1% by volume normal goat serum, 0.05% by volume Tween® 20 in PBS was added to each well and incubated at 37°C for another 15 minutes.

The wells were washed five times with 0.05% by volume Tween® 20 in PBS to remove unbound antibody and reacted with 100 µl of the substrate mixture containing 0.04% by weight orthophenylenediamine (OPD) and 0.012% by volume hydrogen peroxide in sodium citrate buffer, pH 5.0. This substrate mixture was used to detect the peroxidase label by forming a colored product. Reactions were stopped by the addition of 100 µl of 1.0M H₂SO₄ and the absorbance measured using an ELISA reader at 492nm (i.e. A₄₉₂). Assays were performed in duplicate with one dilution (1:20) of serum samples from normal individuals or from patients with diseases unrelated to HTLV-I infection used as negative controls. Absorbance readings greater than the cutoff value of A₄₉₂ = 0.12, (about 3x the mean A₄₉₂ value of normal serum control), were taken as positive. The results are shown in Fig. 3.

### EXAMPLE 2

### Detection of Antibodies to HTLV by an Enzyme-Linked Immunoadsorbent Assay

Wells of 96-well plates were coated at 4°C overnight (or for 3 hours at room temperature or for 1 hour at 37°C), with a mixture of four peptides prepared as described in a ratio by weight of II:IV:V:VI = 1:0.25:1:1 at 3.25 µg per well of the mixture in 100 µl 10mM NaHCO₃ buffer, pH 9.5. The wells were washed three times with phosphate buffered saline (PBS) and then incubated with 250 ul of 3% by weight of gelatin in PBS at 37°C for 1 hour to block non-specific protein binding sites, followed by three more washes with PBS containing 0.05% by volume of Tween® 20. The test sera (blood taken from human patients or normal individuals) were diluted with PBS containing 20% by volume normal goat serum, 1% by weight gelatin and 0.05% by volume Tween® 20 at dilutions of 1:20, volume to volume, respectively. 200 µl of the diluted sera were added to each well and allowed to react for 1 hour at 37° C. The wells were then washed three times with 0.05% by volume Tween® 20 in PBS in order to remove unbound antibodies. Horseradish peroxidase conjugated goat anti-human IgG was used as a second antibody tracer to bind with the HTLV antibody-antigen complex formed in positive wells. 100 µl of peroxidase labeled goat anti human IgG at a dilution of 1:3000 in 1% by volume normal goat serum, 0.05% by volume Tween® 20 in PBS was added to each well and incubated at 37°C for another 15 minutes.

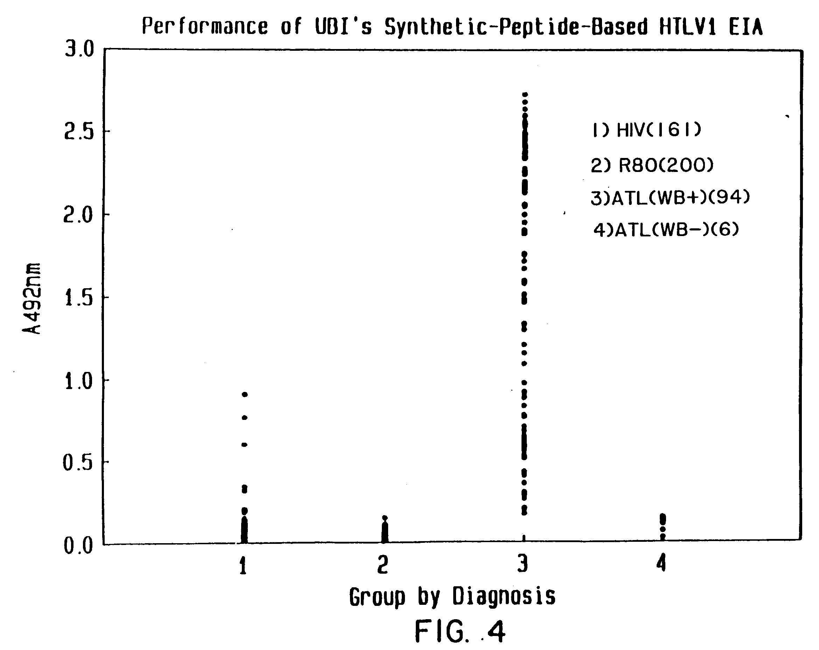

The wells were washed five times with 0.05% by volume Tween® 20 in PBS to remove unbound antibody and reacted with 100 µl of the substrate mixture containing 0.04% by weight orthophenylenediamine (OPD) and 0.012% by volume hydrogen peroxide in sodium citrate buffer, pH 5.0. This substrate mixture was used to detect the peroxidase label by forming a colored product. Reactions were stopped by the addition of 100 µl of 1.0M H₂SO₄ and the absorbance measured using an ELISA reader at 492nm (i.e. A₄₉₂). Assays were performed in duplicate with one dilution (1:20) of serum samples from normal individuals or from patients with diseases unrelated to HTLV infection used as negative controls. Absorbance readings greater than the cutoff value of A₄₉₂ = A₄₉₂ value for normal control + 0.1 (A₄₉₂ value for a reactive control), were taken as positive. The results are shown in Table I and Figure 4.

**TABLE I**

| Detection of Antibodies to HTLV by ELISA* Using a Mixture of Four Peptides as Solid Phase Immunoadsorbent | | | |
|---|---|---|---|
| | Subject | No. Positive/ No. Tested* | Percent Positive |
| 1. | Patients (Lot 5) with ATL (HTLV Western Blot Positive) | 94/94 | 100 |
| 2. | Patients (Lot 5) with ATL (HTLV Western Blot Negative) | 0/6 | 0 |
| 3. | Patients with AIDS/ARC or known to be infected with HIV | 10/161 | 6 |
| 4. | Normal Subjects | 0/200 | 0 |

| | | | |
|---|---|---|---|
| *Assay was performed using sera at 1:20 (v/v) dilution with buffer. | | | |
| Note: Sera from patients with ATL were kindly provided by the Japanese Red Cross, sera from patients with AIDS, ARC, Primary Immunodeficiency, Leukemia/Lymphomas were kindly provided by Dr. S. Gupta at the University of California at Irvine, Dr. D. M. Knowles at Columbia University, and Dr. F. D. Siegal at the Long Island Jewish Hospital. | | | |

The results in Table I show that the ELISA test procedure according to the present invention with sera samples is very accurate and highly specific. No immunoreactivity was found in sera from normal subjects.

It is to be noted that in screening tests to exclude virus contaminated blood from blood banks, the criteria for defining positive reactions may be made more stringent if desired.

### EXAMPLE 3

### Detection Of Antibodies to HTLV By An Agglutination Assay

The presently claimed HTLV peptides, synthesized according to the Merrifield solid phase method, were conjugated to bovine serum albumin (BSA) which had been derivatized with m-maleimidobenzoyl-N-hydroxysuccinimide ester (MBS), essentially as described by Fu-Tong Liu et al, in Biochemistry 18:690-697 (1979). To 0.32 ml. of a BSA solution (100 mg/ml in 0.01 M phosphate buffer, pH 7.0) at room temperature was added 0.013 ml of an MBS solution (0.025 mg/ml in dimethylformamide). The amount of MBS added to the BSA solution can be varied according to the optimal molar ratio of BSA to MBS determined for a specific conjugate studied. The mixture was stirred at room temperature for 1 hour, after which it was centrifuged to remove any precipitated albumin. The clarified mixture was then subjected to gel filtration on Sephadex® G-25 and the protein-containing fractions, as detected by their absorbance at 280 nm, were pooled and stored frozen at -70°C until needed.

The peptides were dissolved in H₂O at 10 mg/ml. A predetermined amount of each peptide solution was added dropwise to the previously activated BSA-MBS solution and stirred at room temperature for 3 hours. The final peptide-BSA conjugates were separated from other free peptides by gel filtration or extensive dialysis. The ratio of peptide to BSA was determined by SDS-PAGE according to conventional methods.

In one example, conjugated peptide VI-BSA was then adsorbed to double aldehyde fixed human O erythrocytes at pH 4.0. The peptide-conjugate coated erythrocytes were then treated with NaBH₄ to prevent non-specific protein binding. The peptide-conjugate coated erythrocytes were then washed with PBS and incubated with 5% normal human serum-PBS solution. These processed cells were then used in an agglutination assay for the detection of HTLV antibodies in both serum and plasma specimens.

A total of 100 sera from patients with adult T cell leukemia were tested for antibodies to HTLV by (1) an enzyme immunoassay (EIA) employing HTLV-I viral lysate as the solid phase [DuPont's HTLV-I ELISA]; (2) the Western Blot (WB) analysis; (3) the above-described HTLV agglutination assay employing peptide VI-BSA conjugate as the solid phase.

The results are shown in Table II.

**TABLE II**

| Results | | | |
|---|---|---|---|
| WB | No. Tested | EIA | HTLV Agglutination Assay |
| + | 77 | + | 77 positive+ |
| indeterm. | 2 | + | 2 negative* |
| - | 21 | - | 21 negative |

| | | | |
|---|---|---|---|
| * The two specimens that tested negative with the HTLV agglutination assay were found to have antibodies only to the p19 core protein of HTLV. | | | |

### EXAMPLE 4

### Simultaneous Detection Of Antibodies To HTLV and HIV (1 and 2) By An Enzyme Immunoassay Employing A Mixture Of Seven Chemically Synthesized Peptides

A solution containing seven of the chemically synthesized peptides of the present invention was used to coat the wells of 96 well plates, according to the procedure of Example I. Three of the peptides were derived from the HTLV-I peptide family [II, IV and VI]; three, from the HIV-1 peptide family [XIII, XIV and XV]; and one, from the HIV-2 peptide family [XVI]. The peptides II:IV::VI:XIII:XIV:XV:XVI were present at a ratio of 2:0.2:2:10:1:1:5 for a total concentration of 21.2 µg/ml. A total of 771 specimens from donors known to be HIV-1 positive (155 specimens); HIV-2 positive (10 specimens); HTLV positive by Western Blot (92 specimens); HTLV negative by Western Blot (4 specimens); patients with autoimmune diseases (AI, 36 specimens); and, from random blood donors (RBD, 474 specimens), were tested on the peptide-coated plates for their respective retroviral immunoreactivities.

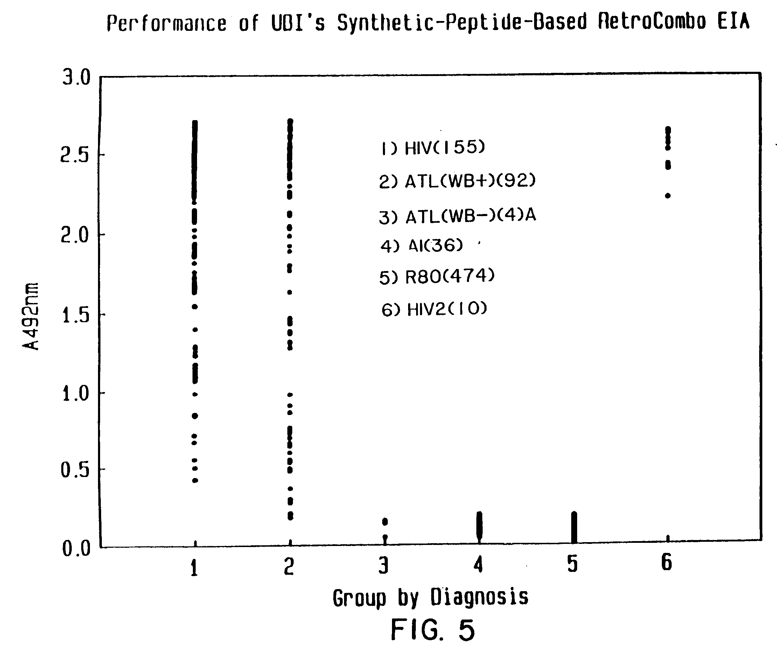

Performance of this synthetic peptide-based retroviralcombo EIA (HTLV and HIV-1 and 2) with these specimens is illustrated in Figure 5. The results clearly indicate the usefulness of these HTLV peptides in conjunction with the HIV peptides for the detection of retroviral infections.

### EXAMPLE 5

### Detection of Antibodies to HTLV by an Immunoradiometric Assay (IRMA)

Wells of 96-well flexible-polyvinylchloride (PVC) plates are coated at 4°C overnight (or 3 hours at room temperature) with a mixture (1:1:1) of these three peptides, prepared as described, at 1.5 µg per well in 100 µl 10mM NaHCO₃ suffer, pH 9.5. The wells are washed three times with phosphate buffered saline (PBS) and then incubated with 250 µl of 3% by weight gelatin in PBS at 37°C for 1 hour to block non-specific protein binding sites, followed by three more washes with PBS containing 0.05% by volume Tween® 20. The test sera (blood taken from a human patient or normal individual) are diluted with PBS containing 20% by volume normal goat serum, 1% by weight gelatin and 0.05% by volume Tween 20 at dilutions of 1:20 and 1:200 (volume to volume) respectively. 200 µl of the diluted sera are added to each cell and allowed to react for 1 hour at 37°C. The wells are then washed three times with 0.05% by volume Tween® 20 in PBS in order to remove unbound antibodies. I-125 labeled affinity purified goat antihuman IgG is used as a second antibody tracer that binds with the antibody-antigen complex formed in positive wells. 100 ul of I-125 labeled goat antihuman IgG of 50,000-200,000 cpm in 1% by volume normal goat serum, 0.05% by volume Tween® 20 in PBS is added to each well and incubated at 37°C for another hour.

The wells are washed five times with 0.05% by volume Tween® 20 in PBS to remove unbound second antibody and dried. The wells are cut and counted by a gamma-scintillation counter. Assays are performed in duplicate with a 1:20 dilution volume to volume. Normal sera sample as negative controls are also tested simultaneously. Cpm readings greater than the average readings of normal sera samples + 4SD (standard deviation) are taken as positive.

### EXAMPLE 6

### Detection Of Antibodies To HTLV By An Agglutination Assay Utilizing As The Solid Phase. Immunoadsorbent Gelatin Particles, Erythrocytes Of Different Animal Species, Or Latex Beads Coated With A Mixture Of Peptides

One ml thoroughly washed erythrocytes, gelatin particles, or polystyrene latex beads are coated with the peptide mixture at concentration in the range of 5 µg/ml to 1 mg/ml. The peptide mixture coated cells, particles or beads are then incubated with serially diluted serum samples in the wells of a 96-well U-shaped microplate or on a slide. After being left at room temperature for about an hour, the settled agglutination patterns on the bottom of the wells or on the slide are read, and the largest dilution showing a positive reaction is recorded.

This is a one-step assay which could be used for both qualitative and quantitative analysis for the presence of antibodies to HTLV in specimens including sera or biofluids.

### EXAMPLE 7

A third test kit for detecting HTLV antibodies using the agglutination assay comprises a compartmented enclosure containing multiple microwell plates arid other accessory materials for an agglutination assay including (1) a bottle of peptide mixture coated erythrocytes, gelatin particles or latex polystyrene beads; (2) normal human serum (as a negative control); and, (3) NP40 treated-and heat inactivated, HTLV-1 seropositive serum (as a positive control), and (4) specimen diluent. The procedure described in Example 3 is to be followed.

### EXAMPLE 8

A diagnostic test kit for the detection of HTLV antibodies can be constructed. The test kit comprises a compartmented enclosure containing multiple 96-well plates coated prior to use with the peptide(s) or peptide mixture(s) of the present invention in 100 ul pH 9.5 10mM NaHCO₃ buffer. The kit further comprises materials for enzyme detection in separate sealed containers consisting of: 1) normal human serum (as a negative control); 2) NP40 treated and heat inactivated, HTLV-I seropositive serum (as a positive control); 3) specimen diluent; 4) peroxidase labeled-goat antihuman IgG; and 5) a color change indicator consisting of, for example, orthophenylenediamine (OPD) and hydrogen peroxide in phosphate citrate buffer. The procedure described in Example 1 is to be followed.

In this test kit, 96-well plates, precoated with a peptide or peptide mixture of the present invention, can be replaced by polystyrene beads, or multiple mini-columns filled with controlled pore size glass beads, or nitrocellulose paper strip precoated with the peptides of the present invention for use as the solid phase immunoadsorbent.

### EXAMPLE 9

A second test kit for detecting antibodies using the immunoradiometric assay (IRMA) comprises a compartmented enclosure containing multiple 96-well bendable polyvinylchloride (PVC) plates precoated with the peptide(s) or peptide mixture(s) according to the present invention in 100 µl of pH 9.5 10mM NaHCO₃ buffer and materials for radioiommunoassay including: 1) normal human serum (as a negative control); 2) NP40 treated and heat inactivated, HTLV-1 seropositive serum (as a positive control); 3) specimen diluent; and, 4) I-125 labeled goated anti human IgG. The procedure described in Example 5 is to be followed.

In this test kit, 96-well PVC plates precoated with the peptides of the present invention can be replaced by polystyrene beads precoated with the peptide of the present invention for use as the solid phase immunoadsorbent.

### EXAMPLE 10

### Specific Detection of Antibodies to HTLV-I and not HTLV-II By An-Enzyme Immunoassay Employing A Synthesized Peptide

A solution containing the synthesized HTLV-I peptide (IV) of the present invention at 5 µg/ml having the sequence corresponding to HTLV-I, APPLLPHSNLDHILEPSIPWKSKLLTLVQLTLQS, was used to coat the wells of 96 well plates, according to the procedure of Example 1. A total of 120 specimens from blood donors or individuals known to be repeat reactive on an HTLV lysate based test were tested on the peptide-coated plates for their immunoreactivity. Of the 120 samples, 73 had also been tested by polymerase chain reaction with HTLV-I or HTLV-II specific DNA probes (PCR). Of these, 43 were positive for HTLV-I by PCR and 30 were positive for HTLV-II by PCR. Supplemental testings, such as Western Blot and radioimmunoprecipitation assay (RIPA), were also performed on all 120 samples. For those samples with no PCR results available, the WB and RIPA results were considered as probably HTLV-I or HTLV-II positive. The 120 samples thus comprised the following categories: HTLV-I positive by PCR (43 specimens); probably HTLV-I positive by Western Blot and RIPA (12 specimens); HTLV-II positive by PCR (30 specimens); probably HTLV-II positive by Western Blot and RIPA (26 specimens); and repeat reactive for HTLV by viral lysate ELISA, but negative by Western blot and negative by RIPA (RR (WB NEG), 9 specimens).

Performance of this synthetic peptide-based EIA (HTLV-I specific) is presented in Table III. The results in Table III show that the method is highly sensitive and specific for HTLV-I and that it can be used to distinguish HTLV-II from HTLV-I infection. The whole virus lysate EIA, on the other hand, does not distinguish between the two viral infections since it gave positive results for all 120 samples.

**TABLE III**

| TESTED BY PEPTIDE IV | | | |
|---|---|---|---|
| | Subject (confirmed by) | No. Positive/ No. Tested | Percent Positive |
| 1. | HTLV-I (PCR) | 41/43 | 95.3 |
| 2. | HTLV-I (WB/RIPA) | 12/12 | 100 |
| 3. | HTLV-II (PCR) | 4/30 | 13.3 |
| 4. | HTLV-II (WB/RIPA) | 1/26 | 3.8 |
| 5. | RR (WB NEG) | 0/9 | 0 |

Sera from blood donors and individuals known to be HTLV-I or HTLV-II positive, confirmed either by PCR, RIPA or WB, were kindly provided by Serologicals, Inc. and by Dr. Chang Fang of the American Red Cross (ARC).

### EXAMPLE 11

### Specific Detection of Antibodies to HTLV-II and not HTLV-I By An Enzyme Immunoassay Employing A Synthesized Peptide

A solution containing a synthetic peptide analogue of the HTLV-I peptide IV of the present invention, designated as HTLV-II peptide X, having the sequence corresponding to HTLV-II in Figure 2, i.e., with an amino acid sequence of SPPLVHDSDLEHVLTPSTSWTTKILKFIQLTLQS, was used at a concentration of 5ug/ml to coat the wells of 96 well plates, according to the procedure of Example 1. The same 120 specimens as in Example 10 were tested.

Performance of this synthetic peptide-based EIA (HTLV-II specific) is presented in Table IV. The results in Table IV show that the method is highly sensitive and specific for HTLV-II and that it can be used to distinguish antibodies against HTLV-II from antibodies against HTLV-I. The whole virus lysate EIA, on the other hand, does not distinguish between the two viral infection since it gave positive results for all 120 samples.

**TABLE IV**

| TESTED BY PEPTIDE X | | | |
|---|---|---|---|
| | Subject (Confirmed by) | No. Positive/ No. Tested | Percent Positive |
| 1. | HTLV-II (PCR) | 28/30 | 93.3 |
| 2. | HTLV-II (WB/RIPA) | 24/26 | 92.3 |
| 3. | HTLV-I (PCR) | 0/43 | 0 |
| 4. | HTLV-I (WB/RIPA) | 0/12 | 0 |
| 5. | RR (WB NEG) | 0/9 | 0 |

It is to be understood that the above examples are illustrative of the present invention and are not meant to limit the scope thereof.

### REFERENCES

1. B.J. Poiesz., et al., Proc. Natl Acad. Sci. USA., 77:7415 (1980).
2. B. J. Poiesz., F.W. Ruscetti,M., S. Reitz., V.S.Kalyanaraman, R. Gallo, Nature (London) 294:268 (1981).
3. R.C. Gallo et al., Proc. Natl. Acad. Sci. USA., 79:5680 (1982).
4. M.Essex et al., Science, 221:1061 (1983).
5. P. Clapham, K. Napy, R. A. Weiss, Proc. Natl. Acad. Sci. 81:2886 (1984).
6. R. C. Gallo et al., Cancer Res., 43: 3892 (1983).
7. R. C. Gallo, Cancer Surveys, L. M. Franks et al. Eds, (University Press, Oxford, in press).
8. W. A. Blattner, K. Tokatsuki, R. C. Gallo. J.Am. Med. Assoc., 250:1074 (1983).
9. K. Takatsuki, J. Uchiyama, K. Sagawa, J. Yodoi, Topics in Hematology, S. Seno, F. Takaku, S. Irino, Eds.(Excerpta Medica, Amsterdam, 1977) p73.
10. W. A. Blattner et al., Int. J. Cancer, 30:257 (1982)
11. D. Catovsky et al., Lancet, 1982-I, 639 (1982).
12. D. W. Blayney et al., J. Am. Med. Assoc., 250:1048 (1983).
13. M. Robert-Guroff, F. W. Ruscetti, L. W. Posner, B. J.Poiesz, R. C. Gallo, J. Exp. Med., 154: 1957 (1981).
14. R. C. Gallo et al., Proc. Natl. Acad. Sci. USA., 79:5680 (1981).
15. M. Robert-Guroff et al., J. Exp. Med., 157:248 (1983).
16. M. Shimoyama et al, Jpn. J. Clin. Oncol., 12:109 (1982).
17. M. Seiki, S. Hattori, Y. Hirayama, M. Yoshida Proc. Natl Acad. Sci. USA., 80:3618 (1983).
18. Saxinger, C. W. et al., Science, 225:1473 (1984).
19. Samuel, K.P. et al., Science, 226, 1094-1097 (Nov. 30, 1984).
20. Slamon et al., PCT Patent Publication No. W086/01834.
21. Wang, J.J-G, Steel, S., Wisniewolski, R. and Wang, C.Y. Proc. Natl. Acad. Sci. USA, 83, pp 6159-6163 (August 1986).
22. U.S. Patent No. 4,735,896. issued April 5, 1988 to Chang Y. Wang and James G. Wang.
23. U.S. Patent No. 4,879,212 issued Nov. 7, 1989 to Chang Y. Wang and James G. Wang.
24. Liu, Fu-Tong et al., Biochemistry, 18, pp. 690-697 (1979).
25. V.S. Kalyanaraman, M.G. Sarngadharan, M. Robert-Guroff et al., Science, 218: 571 (1982)
26. J.D. Rosenblatt et al., N. Engl. J. Med., 315:372 (1986).
27. M. Robert-Guroff, S.H. Weiss, J.H. Giron et al., J. Am. Med. Assoc., 255:3133 (1986).
28. H. Lee, P. Swanson, V.S. Shorty et al., Science, 244:471 (1989).
29. R.C. Gallo, Med. Oncol. Tumor Pharmacother., 3:265 (1986).
30. K. Shimotolino, Y. Takahashi, et al., PNAS USA, 82, 3101-3105 (May 1985).
31. J. Sodroski, R. Patarca, D. Perkins et al., Science, 225:421 (1984).
32. G.M. Shaw, M.A. Gonda, G.H. Flickinger et al., PNAS USA, 81:4544 (1984).
33. S.G. Sandler, C. Fang, in Transfusion-Transfmitted Viral Disease, Arlington, VA:American Assn. of Blood Bankers, p. 19 (1987)

## Claims

1. A peptide composition having specific immunoreactivities to antibodies of HTLV comprising a peptide:
APPLLPHSNLDHILEPSIPWKSKLLTLVQLTLQS-X (IV)
alone or in a mixture with any or both of
SSTPLLYPSLALPAPHLTLPFNWTHCFDPQIQAIVSSPCH-X (V)
or
CFDPQIQAIVSSPCHNSLILPPFSLSPVPTLGSRSRRA-X (VI)
wherein X is -OH or -NH₂, analogues of said peptides having an amino acid sequence derived from a strain/isolate of HTLV in a region corresponding to the peptide and having specific immunoreactivity to antibodies to HTLV and conjugates and polymers of the peptide.

2. A peptide composition according to claim 1 comprising a mixture of the peptides IV, V and VI.

3. A peptide composition according to claim 1 further comprising a peptide selected from the group consisting of:
GLDLLFWEQGGLCKALQEQC-X (I)
QNRRGLDLLFWEQGGLCKALQEQC-X (II)
NRRGLDLLFWEQGGLC-X (III)
wherein X is -OH or NH₂, analogues of said peptides having an amino acid sequence derived from a strain/isolate of HTLV in a region corresponding to the peptide and having specific immunoreactivity to antibodies to HTLV and conjugates and polymers of the peptides.

4. A peptide composition according to claim 1 wherein the analogues respectively of peptide IV, V and VI are:
SPPLVHDSDLEHVLTPSTSWTTKILKFIQLTLQS-X (X)
SSRTILFPSLALPAPPSQPSLWTHCYQPRLQAITTDNCN-X (XI)
CYQPRLQAITTDNCNNSIILPPFSLAPVPLATRRRRA-X (XII)

5. An immunoassay method for the detection of antibodies to HTLV comprising:
A. coating a solid support with an effective amount of a peptide composition comprising a peptide selected from the group consisting of:
APPLLPHSNLDHILEPSIPWKSKLLTLVQLTLQS-X (IV)
alone or in a mixture with any or both of
SSTPLLYPSLALPAPHLTLPFNWTHCFDPQIQAIVSSPCH-X (V)
or
CFDPQIQAIVSSPCHNSLILPPFSLSPVPTLGSRSRRA-X (VI)
wherein X is -OH or -NH₂, analogues of said peptides having an amino acid sequence derived from a strain/isolate of HTLV in a region corresponding to the peptide and having specific immunoreactivity to antibodies to HTLV and conjugates and polymers of the peptide;
B. mixing a test specimen diluted with a buffer wherein the antibodies to HTLV in the test specimen form a peptide-antibody complex with the peptide;
C. incubating the mixture; and
D. detecting the presence of the peptide-antibody complex.

6. The immunoassay method according to claim 5 wherein step D comprises: introducing a second known antibody labeled with an enzyme and a substrate which reacts with the enzyme to form a colored product.

7. The method according to claim 5, wherein the peptide is an analogue respectively of peptides IV, V and VI having the amino acid sequences:
SPPLVHDSDLEHVLTPSTSWTTKILKFIQLTLQS-X (X)
SSRTILFPSLALPAPPSQPSLWTHCYQPRLQAITTDNCN-X (XI)
CYQPRLQAITTDNCNNSIILPPFSLAPVPLATRRRRA-X (XII)

8. The method according to claim 6 wherein the peptide composition is an effective amount of a mixture of II:IV:V:VI.

9. The immunoassay method according to claim 5 wherein step D comprises: introducing a second known antibody labelled with a radioactive element.

10. The immunoassay method according to claim 5 wherein the peptide-antibody complex is detectable as an agglutination pattern.

11. The immunoassay method according to claim 5 wherein the solid support is a strip coated with the peptide composition in a multidot multi-line or multi-square array.

12. A test kit for the detection of antibodies to HTLV and the diagnosis of HTLV infection comprising:
a. a solid support;
b. coated onto the solid support an immunoadsorbent comprising a peptide composition according to claim 1;
c. a sample of normal serum as negative control;
d. a sample of serum containing antibodies to HTLV as positive control; and
e. a buffer for diluting the serum samples.

## Patentansprüche

1. Peptidzusammensetzung mit spezifischer Immunreaktivität gegen Antikörper von HTLV, enthaltend ein Peptid:
APPLLPHSNLDHILEPSIPWKSKLLTLVQLTLQS-X (IV)
allein oder in einer Mischung mit einem oder beiden von
SSTPLLYPSLALPAPHLTLPFNWTHCFDPQIQAIVSSPCH-X (V)
oder
CFDPQIQAIVSSPCHNSLILPPFSLSPVPTLGSRSRRA-X (VI),
worin X -OH oder -NH₂, Analoge der Peptide mit einer von einem Stamm/Isolat von HTLV in einer dem Peptid entsprechenden Region abgeleiteten Aminosäuresequenz und mit einer spezifischen Immunreaktivität gegen Antikörper von HTLV, sowie Konjugate und Polymere des Peptids sind.

2. Peptidzusammensetzung nach Anspruch 1, enthaltend eine Mischung der Peptide IV, V und VI.

3. Peptidzusammensetzung nach Anspruch 1, weiter enthaltend ein aus der Gruppe bestehend aus:
GLDLLFWEQGGLCKALQEQC-X (I)
QNRRGLDLLFWEQGGLCKALQEQC-X (II)
NRRGLDLLFWEQGGLC-X (III)
ausgewähltes Peptid,
worin X -OH oder NH₂, Analoge der Peptide mit einer von einem Stamm/Isolat von HTLV in einer dem Peptid entsprechenden Region abgeleiteten Aminosäuresequenz und mit einer spezifischen Immunreaktivität gegen Antikörper von HTLV, sowie Konjugate und Polymere der Peptide sind.

4. Peptidzusammensetzung nach Anspruch 1, worin die Analoge der Peptide IV, V beziehungsweise VI
SPPLVHDSDLEHVLTPSTSWTTKILKFIQLTLQS-X (X)
SSRTILFPSLALPAPPSQPSLWTHCYQPRLQAITTDNCN-X (XI)
CYQPRLQAITTDNCNNSIILPPFSLAPVPLATRRRRA-X (XII)
sind.

5. Immunoassay-Verfahren zum Nachweis von Antikörpern gegen HTLV enthaltend:
A. Beschichten eines festen Trägers mit einer wirksamen Menge einer Peptidzusammensetzung enthaltend ein aus der Gruppe bestehend aus:
APPLLPHSNLDHILEPSIPWKSKLLTLVQLTLQS-X (IV)
allein oder in einer Mischung mit einem oder beiden von
SSTPLLYPSLALPAPHLTLPFNWTHCFDPQIQAIVSSPCH-X (V)
oder
CFDPQIQAIVSSPCHNSLILPPFSLSPVPTLGSRSRRA-X (VI)
ausgewähltes Peptid,
worin X -OH oder -NH₂, Analoge der Peptide mit einer von einem Stamm/Isolat von HTLV in einer dem Peptid entsprechenden Region abgeleiteten Aminosäuresequenz und mit einer spezifischen Immunreaktivität gegen Antikörper von HTLV, sowie Konjugate und Polymere des Peptids sind;
B. Mischen einer mit einem Puffer verdünnten Testprobe, worin die Antikörper gegen HTLV in der Testprobe mit dem Peptid einen Peptid-Antikörper-Komplex bilden;
C. Inkubieren der Mischung und
D. Nachweis des Vorliegens des Peptid-Antikörper-Komplexes.

6. Immunoassay-Verfahren nach Anspruch 5, worin der Schritt D die Einführung eines zweiten, bekannten mit einem Enzym markierten Antikörpers und einem Substrat umfaßt, das mit dem Enzym unter Bildung eines gefärbten Produktes reagiert.

7. Verfahren nach Anspruch 5, worin das Peptid ein Analogon der Peptide IV, V beziehungsweise VI mit den Aminosäuresequenzen:
SPPLVHDSDLEHVLTPSTSWTTKILKFIQLTLQS-X (X)
SSRTILFPSLALPAPPSQPSLWTHCYQPRLQAITTDNCN-X (XI)
CYQPRLQAITTDNCNNSIILPPFSLAPVPLATRRRRA-X (XII)
ist.

8. Verfahren nach Anspruch 6, worin die Peptidzusammensetzung eine wirksame Menge einer Mischung von II : IV : V: VI ist.

9. Immunoassay-Verfahren nach Anspruch 5, worin der Schritt D die Einführung eines zweiten, bekannten mit einem radioaktiven Element markierten Antikörpers umfaßt.

10. Immunoassay-Verfahren nach Anspruch 5, worin der Peptid-Antikörper-Komplex als Agglutionationsmuster nachweisbar ist.

11. Immunoassay-Verfahren nach Anspruch 5, worin der feste Träger ein mit der Peptidzusammensetzung in einer mehrreihigen Anordnung aus vielen Punkten oder aus vielen Quadraten beschichteter Streifen ist.

12. Test-Kit zum Nachweis von Antikörpern gegen HTLV und zur Diagnose einer HTLV-Infektion enthaltend:
a. einen festen Träger,
b. ein auf den festen Träger aufbrachtes Immunadsorbens mit einer Peptidzusammensetzung nach Anspruch 1,
c. eine Probe eines Normalserums als Negativkontrolle;
d. eine Probe eines Antikörpers gegen HTLV enthaltenden Serums als Positivkontrolle
und
e. einen Puffer zur Verdünnung der Serumproben.

## Revendications

1. Composition peptidique ayant des immunoréactivités spécifiques avec les anticorps de HTLV et comprenant un peptide :
APPLLPHSNLDHILEPSIPWKSKLLTLVQLTLQS-X (IV)
seul ou en mélange avec l'un des peptides ou les deux de formules :
SSTPLLYPSLALPAPHLTLPFNWTHCFDPQIQAIVSSPCH-X (V)
ou
CFDPQIQAIVSSPCHNSLILPPPSLSPVPTLGSRSRRA-X (VI)
où X est -OH ou -NH₂, les analogues desdits peptides ayant une séquence aminoacide dérivée d'une souche/isolat de HTLV dans une région correspondante du peptide et ayant une immunoréactivité spécifique avec les anticorps de HTLV et les conjugués et polymères du peptide.

2. Composition peptidique suivant la revendication 1, comprenant un mélange des peptides IV, V et VI.

3. Composition peptidique suivant la revendication 1, comprenant en outre un peptide choisi parmi l'ensemble constitué par :
GLDLLFWEQGGLCKALQEQC-X (I)
QNRRGLDLLFWEQGGLCKALQEQC-X (II)
NRRGLDLLFWEQGGLC-X (III)
où X est -OH ou -NH₂, les analogues desdits peptides ayant une séquence aminoacide dérivée d'une souche/isolat de HTLV dans une région correspondante du peptide et ayant une immunoréactivité spécifique avec les anticorps de HTLV et les conjugués et polymères du peptide.

4. Composition peptidique suivant la revendication 1, dans laquelle les analogues des peptides IV, V et respectivement VI sont :
SPPLVHDSDLEHVLTPSTSWTTKILKFIQLTLQS-X (X)
SSRTILFPSLALPAPPSQPSLWTHCYQPRLQAITTDNCN-X (XI)
CYQPRLQAITTDNCNNSIILPPFSLAPVPLATRRRRA-X (XII)

5. Procédé immunologique pour la détection d'anticorps de HTLV, ledit procédé comprenant :
A. le revêtement d'un support solide avec une quantité efficace d'une composition peptidique comprenant un peptide choisi parmi l'ensemble constitué par :
APPLLPHSNLDHILEPSIPWKSKLLTLVQLTLQS-X (IV)
seul ou en mélange avec l'un des peptides ou les deux de formules :
SSTPLLYPSLALPAPHLTLPFNWTHCFDPQIQAIVSSPCH-X (V)
ou
CFDPQIQAIVSSPCHNSLILPPFSLSPVPTLGSRSRRA-X (VI)
où X est -OH ou -NH₂, les analogues desdits peptides ayant une séquence aminoacide dérivée d'une souche/isolat de HTLV dans une région correspondante du peptide et ayant une immunoréactivité spécifique avec les anticorps de HTLV et les conjugués et polymères du peptide ;
B. l'ajout d'un échantillon à tester dilué au moyen d'un tampon de façon que les anticorps de HTLV de l'échantillon à tester puissent former un complexe peptide-anticorps avec le peptide ;
C. l'incubation du mélange résultant ; et,
D. la détection de la présence du complexe peptide-anticorps.

6. Procédé immunologique suivant la revendication 5, dans lequel l'étape D comprend l'introduction d'un second anticorps connu marqué avec un enzyme et d'un substrat qui réagit avec l'enzyme pour former un produit coloré.

7. Procédé immunologique suivant la revendication 5, dans lequel le peptide est l'un des analogues des peptides IV, V et, respectivement, VI ayant les séquences aminoacides :
SPPLVHDSDLEHVLTPSTSWTTKILKFIQLTLQS-X (X)
SSRTILFPSLALPAPPSQPSLWTHCYQPRLQAITTDNCN-X (XI)
CYQPRLQAITTDNCNNSIILPPFSLAPVPLATRRRRA-X (XII)

8. Procédé suivant la revendication 6, dans lequel la composition peptidique est une quantité efficace d'un mélange de II/IV/V/VI.

9. Procédé immunologique suivant la revendication 5, dans lequel l'étape D comprend l'introduction d'un second anticorps connu marqué avec un élément radioactif.

10. Procédé immunologique suivant la revendication 5, dans lequel le complexe peptide-anticorps est détectable selon une technique d'agglutination.

11. Procédé immunologique suivant la revendication 5, dans lequel le support solide est un ruban revêtu de la composition peptidique selon un arrangement dit à plusieurs points, à plusieurs lignes ou à plusieurs pavés.

12. Nécessaire pour la détection d'anticorps de HTLV et le diagnostic d'une infection due à HTLV, ledit nécessaire comprenant :
a. un support solide ;
b. un immunoabsorbant revêtant le support et comprenant une composition peptidique selon la revendication 1 ;
c. un échantillon de sérum normal en tant que témoin négatif ;
d. un échantillon de sérum contenant des anticorps de HTLV en tant que témoin positif ; et
e. un tampon pour diluer les échantillons.
